# EUROPÄISCHE PATENTSCHRIFT

(11) **EP 2 327 447 B1**
(45) Veröffentlichungstag und Bekanntmachung des Hinweises auf die Patenterteilung: **30.05.2012**
(21) Anmeldenummer: 10187874.2
(22) Anmeldetag: 18.10.2010
(51) Int. Cl.: A61N 1/368, A61N 1/365

(54) **Biventrikulärer Herzstimulator**
Biventricular cardiac stimulator
Stimulateur cardiaque bi-ventriculaire

(30) Priorität: 09.11.2009 US 259228 P
(43) Veröffentlichungstag der Anmeldung: 01.06.2011
(73) Patentinhaber: Biotronik CRM Patent AG, 6341 Baar (CH)
(72) Erfinder: Vollkron, Michael, 3021, Pressbaum (AT); Krämer, Thomas, 90425, Nürnberg (DE); Lippert, Michael, 91522, Ansbach (DE); Czygan, Gerald, 91054, Buckenhof (DE)
(74) Vertreter: Lindner-Vogt, Karin L.

(56) Entgegenhaltungen:
- EP-A1- 1 977 784
- US-A1- 2004 158 293
- US-A1- 2008 306 567
- DATABASE MEDLINE [Online] US NATIONAL LIBRARY OF MEDICINE (NLM), BETHESDA, MD, US; November 2006 (2006-11), WHINNETT Z I ET AL: "Haemodynamic effects of changes in atrioventricular and interventricular delay in cardiac resynchronisation therapy show a consistent pattern: analysis of shape, magnitude and relative importance of atrioventricular and interventricular delay.", XP002631641, Database accession no. NLM16709698 & HEART (BRITISH CARDIAC SOCIETY) NOV 2006 LNKD- PUBMED:16709698, Bd. 92, Nr. 11, November 2006 (2006-11), Seiten 1628-1634, ISSN: 1468-201X

## Beschreibung

Die Erfindung betrifft einen biventrikulären Herzstimulator mit einer rechtsventrikulären Sensingeinheit und einer linksventrikulären Sensingeinheit und einem Schrittmacherzeitgeber, der u. a. Zeitpunkte für die Abgabe von Stimulationsimpulsen, beispielsweise rechtsventrikulären oder linksventrikulären Stimulationsimpulsen, bestimmt. Die rechtsventrikuläre Sensingeinheit ist mit einer rechtsventrikulären Wahrnehmungselektrode verbunden oder kann mit einer solchen verbunden werden, während die linksventrikuläre Sensingeinheit entsprechend mit einer linksventrikulären Wahrnehmungselektrode verbunden ist oder mit einer solchen verbunden werden kann.

Biventrikuläre Herzschrittmacher sind typischerweise zur Stimulation des rechten und des linken Ventrikels eines Herzens ausgebildet, um beispielsweise eine kardiale Resynchronisationstherapie (CRT) durchzuführen. Dazu weist ein biventrikulärer Herzstimulator typischerweise eine rechtsventrikuläre Stimulationseinheit und eine linksventrikuläre Stimulationseinheit auf, die jeweils mit wenigstens einer rechtsventrikulären bzw. einer linksventrikulären Stimulationselektrode verbunden oder zu verbinden sind.

Die rechtsventrikuläre(n) Wahrnehmungselektrode(n) und die rechtsventrikuläre(n) Stimulationselektrode(n) sind dabei typischerweise an einer rechtsventrikulären Elektrodenleitung angebracht, während die linksventrikuläre(n) Wahmehmungselektrode(n) und die linksventrikuläre(n) Stimulationselektrode(n) Bestandteile einer linksventrikulären Elektrodenleitung sind. Derartige linksventrikuläre Elektrodenleitungen sind typischerweise dazu vorgesehen, über den Coronar Sinus bis in die Nähe des linken Ventrikels vorgeschoben zu werden und werden daher auch als CS-Elektrodenleitungen bezeichnet.

Ein Schrittmacherzeitgeber bestimmt Zeitpunkte, zu denen Stimulationsimpulse an jeweilige Herzkammern abzugeben sind, auf Basis stimulierter oder wahrgenommener Ereignisse. Ein stimuliertes Ereignis ist die Abgabe eines Stimulationsimpulses, der zu einer Kontraktion der entsprechenden Herzkammer führt. Ein wahrgenommenes Ereignis, auch als natürliches oder intrinsisches Ereignis bezeichnet, ist eine eigenständige Kontraktion der entsprechenden Herzkammer, die über eine entsprechende Wahrnehmungselektrode erfasst wird. Die entsprechende Wahrnehmungselektrode erfasst die mit einer natürlichen Kontraktion einer entsprechenden Herzkammer einhergehenden elektrischen Potenziale. Diese werden verstärkt und durch die Schrittmachersteuerung und insbesondere den Schrittmacherzeitgeber ausgewertet.

In diesem Zusammenhang ist es bekannt, Zeitpunkte für die Abgabe eines jeweiligen Stimulationsimpulses zu bestimmen, die Abgabe aber zu unterdrücken (zu inhibieren), falls innerhalb eines bestimmten Intervalls vor dem vorgesehenen Stimulationszeitpunkt eine eigenständige Kontraktion der jeweiligen Herzkammer, also ein intrinsisches Ereignis dieser Herzkammer, erfasst wird. Wenn der Herzstimulator derart ausgebildet ist, stimuliert er die jeweilige Herzkammer nur bei Bedarf, und daher wird der entsprechende Betriebsmodus auch als Demand-Modus bezeichnet.

Wie bereits erwähnt werden die Zeitpunkte, zu denen ein jeweils nächster Stimulationsimpuls einer jeweiligen Kammer vorgesehen ist, von dem Schrittmacherzeitgeber auf Basis wahrgenommener oder stimulierter Ereignisse bestimmt. In bekannter Manier ist der Schrittmacherzeitgeber hierzu so ausgeführt, dass die Kammern eines Herzens möglichst in einer zeitlichen Reihenfolge kontrahieren, wie es auch ein gesundes Herz selbsttätig in Abhängigkeit des jeweiligen hämodynamischen Bedarfs tut. Beispielsweise folgt auf eine Kontraktion des rechten Atriums nach einer atrioventrikulären Überleitungszeit eine Kontraktion des rechten Ventrikels. Der Schrittmacherzeitgeber bestimmt in analoger Weise den Zeitpunkt für die Abgabe eines nächsten rechtsventrikulären Stimulationsimpulses anhand einer atrioventrikulären Verzögerungszeit (AV-Delay, AVD), die mit einem stimulierten oder erfassten Ereignis im rechten Atrium ausgelöst wird. Zur Festlegung des AVD ist es notwendig, den Zeitpunkt der atrialen Aktivierung oder Kontraktion zu kennen. Dies kann im einfachsten Fall durch Detektion bzw. Stimulation über eine atriale Elektrode erfolgen. Alternativ kann dieser Zeitpunkt beispielsweise auch mit folgenden Methoden bestimmt werden: Analyse von IEGM-Signalen, die zwischen ventrikulären Elektroden und/oder dem Gehäuse gemessen werden (far-field); Analyse von Signalen von Drucksensoren, Volumensensoren oder Flusssensoren, z. B. im rechten Atrium (RA) oder linken Atrium (LA); Analyse von Signalen von Beschleunigungssensoren im oder am RA oder LA.

Vorteilhafterweise ist die atrioventrikuläre Verzögerungszeit derart variabel, dass sich der Schrittmacherzeitgeber möglichst optimal auf einen jeweiligen hämodynamischen Bedarf sowie an die individuellen Bedürfnisse eines jeweiligen Patienten anpassen kann.

Bei einem Herzschrittmacher, der sowohl das rechte Atrium als auch den rechten Ventrikel stimuliert, bestimmt der Schrittmacherzeitgeber auch den Zeitpunkt einer nächsten atrialen Stimulation nach einer VA-Verzögerungszeit, die sich an ein jeweiliges stimuliertes oder erfasstes ventrikuläres Ereignis anschließt und maßgeblich von einer möglichst dem hämodynamischen Bedarf angepassten Herzrate abhängt. Ein Herzstimulator, der einen Schrittmacherzeitgeber besitzt, der die Stimulationsrate an den hämodynamischen Bedarf anpassen kann, wird als ratenadaptiver Herzstimulator bezeichnet.

Bei biventrikulären Herzstimulatoren kann zusätzlich auch der linke Ventrikel stimuliert werden, um die Aktionen (Kontraktionen) des rechten Ventrikels und des linken Ventrikels im Rahmen einer kardialen Resynchronisationstherapie (CRT) zu synchronisieren. In diesem Zusammenhang spielt für den Schrittmacherzeitgeber auch eine interventrikuläre Verzögerungszeit (W-Delay, VVD) eine Rolle, die die zeitliche Verzögerung zwischen der vorgesehenen Abgabe eines rechtsventrikulären Stimulationsimpulses und der vorgesehenen Abgabe eines linksventrikulären Stimulationsimpulses beschreibt und auch Null oder negativ sein kann, so dass beispielsweise auch die Abgabe eines linksventrikulären Stimulationsimpulses vor der Abgabe eines rechtsventrikulären Stimulationsimpulses vorgesehen sein kann. Auch diese interventrikuläre Verzögerungszeit ist vorzugsweise in dem Sinne variabel, dass sich der Schrittmacherzeitgeber auf individuelle Bedürfnisse und momentane Anforderungen bei einem jeweiligen Patienten einstellen kann.

Da die grundsätzliche Funktionsweise biventrikulärer Herzstimulatoren einschließlich derjenigen Herzstimulatoren, die auch das rechte Atrium stimulieren können und daher Drei-Kammer-Stimulatoren sind, als bekannt vorausgesetzt werden kann, kann hier auf eine weitergehende detaillierte Beschreibung verzichtet werden. EP1977784A1 offenbart einen solchen Stimulator mit einem Testmodus zur Optimierung der AVD und VVD. Z.I. Whinnet et. Al beschreiben in "Haemodynamic effects of changes in atrioventricular and interventricular delay show a consistent pattern: analysis of shape, magnitude and relative imporetance of atrioventricular and interventricular delay" (Heart, Nov. 2006, 92: 1628-1634) die Darstellung der AVD und VVD in Matrixform.

Der Erfindung liegt die Aufgabe zugrunde, einen verbesserten Herzstimulator für die kardiale Resynchronisationstherapie zu schaffen.

Ein Herzstimulator gemäß dem Anspruch 1 löst diese Aufgabe.

Der biventrikuläre Herzstimulator weist, eine rechtsventrikuläre Stimulationseinheit auf, die mit einer rechtsventrikulären Stimulationselektrode verbunden oder zu verbinden und ausgebildet ist, auf ein rechtsventrikuläres Triggersignal hin wenigstens einen Stimulationsimpuls über die rechtsventrikuläre Elektrode abzugeben, sowie eine linksventrikuläre Stimulationseinheit, die mit einer linksventrikulären Stimulationselektrode verbunden oder zu verbinden und ausgebildet ist, auf ein linksventrikuläres Triggersignal hin wenigstens einen Stimulationsimpuls über die linksventrikuläre Elektrode abzugeben, und einen Schrittmacherzeitgeber, der mit der rechtsventrikulären Stimulationseinheit und der linksventrikulären Stimulationseinheit verbunden und ausgebildet ist, rechtsventrikuläre Triggersignale und linksventrikuläre Triggersignale am Ende einer atrioventrikulären Verzögerungszeit (AVD) bzw. einer interventrikulären Verzögerungszeit (VVD) abzugeben. Um die Auswirkung einer jeweiligen atrioventrikulären Verzögerungszeit (AVD) und einer jeweiligen interventrikulären Verzögerungszeit (VVD) zu erfassen, besitzt der Herzstimulator eine Erfassungseinheit zum Erfassen eines hämodynamischen Benefits (HDB). Hier und im folgendem sei unter einem HDB entweder ein absoluter Messwert oder aber eine Differenz zu einem Bezugszustand gemeint. Zur Optimierung von AVD und VVD ist der Schrittmacherzeitgeber mit einem Speicher für einen jeweiligen aktuellen Wert für die atrioventrikuläre Verzögerungszeit (AVDₐₖₜ) und die interventrikuläre Verzögerungszeit (VVDₐₖₜ) verbunden und ausgebildet, zu bestimmten Zeitpunkten wenigstens jeweils ein rechtsventrikuläres Triggersignal und ein linksventrikuläres Triggersignal, basierend auf von den aktuellen Werten für die atrioventrikuläre Verzögerungszeit (AVDₐₖₜ) und die interventrikuläre Verzögerungszeit (VVDₐₖₜ) abweichenden Testwerten, für die atrioventrikuläre Verzögerungszeit (AVDₜₑₛₜ) und die interventrikuläre Verzögerungszeit (VVDₜₑₛₜ) auszulösen und den zu einem jeweiligen Wertepaar von AVD_{Test} und VVD_{Test} ermittelten HDB nach Art einer Matrix den beiden Werten für AVD_{Test} und VVD_{Test} zugeordnet zu speichern. Das Speichern des HDB kann dabei auch in mehreren Matrizen erfolgen die basierend auf bestimmten Zuständen z.B: Herzfrequenzbereich, atriales Ereignis, Aktivitätsgrad oder ähnliches ausgewählt werden.

Der HDB ist hierbei durch einen oder mehrere Werte eines oder mehrerer physiologischer Parameter charakterisiert, z. B. den Wert eines Parameters, der sich aus dem Verlauf der intrakardialen Impedanz ergibt. Der jeweilige den HDB beschreibende Wert kann auch ein aus mehreren (Teil-) Werten zusammengesetzter Wert sein.

Geeignete physiologische Parameter können auch von anderen Sensoren abgeleitet werden wie z. B. Druck, Fluss, Volumen, Beschleunigung, Herztöne, Geschwindigkeitsprofile oder ähnliches.

Der erfindungsgemäße Herzstimulator erlaubt eine simultane Optimierung von atrioventrikulärer Verzögerungszeit (AVD) und interventrikulärer Verzögerungszeit (VVD) im laufenden Betrieb eines Herzstimulators für die kardiale Resynchronisationstherapie (CRT). Er verkörpert hierzu einen Nachführungsmechanismus, der beide Parameter so schnell nachführt, dass sie den typischen Änderungen von Belastung, Herzfrequenz, Körperposition, etc. folgen können.

Vorzugsweise ist der Schrittmacherzeitgeber dazu ausgebildet, zur Bestimmung geeigneter Testwerte für die atrioventrikuläre Verzögerungszeit (AVD_{Test}) und die interventrikuläre Verzögerungszeit (VVD_{Test}) gespeicherte Werte für mögliche atrioventrikuläre Verzögerungszeiten und interventrikuläre Verzögerungszeiten und zugehörigen HDB auszuwerten. Besonders bevorzugt ist der Schrittmacherzeitgeber in diesem Zusammenhang dazu ausgebildet, solche Testwerte für die atrioventrikuläre Verzögerungszeit und die interventrikuläre Verzögerungszeit zu wählen, für die noch kein Wert gespeichert ist, der den HDB kennzeichnet, der mit diesen Werten für die atrioventrikuläre Verzögerungszeit und die interventrikuläre Verzögerungszeit verbunden ist.

Alternativ kann der Schrittmacherzeitgeber dazu ausgebildet sein, die aktuell aktive Matrix, oder auch Matrizen die außerhalb des aktuellen Zustandes liegen, nach früheren Ergebnissen zu untersuchen die eine Verbesserung gegenüber dem derzeit Erreichten HDB versprechen und diese für Testzwecke erneut zu Untersuchen und gegebenenfalls deren Parameter auch zu übernehmen.

Alternativ oder zusätzlich kann der Schrittmacherzeitgeber auch dazu ausgebildet sein, solche Testwerte für die atrioventrikuläre Verzögerungszeit und die interventrikuläre Verzögerungszeit auszuwählen, für die ein jeweils zugehöriger, den HDB kennzeichnender Wert zu einem Zeitpunkt bestimmt wurde, der länger als ein vorgegebenes Zeitmaß zurückliegt.

Vorzugsweise ist der Schrittmacherzeitgeber dazu ausgebildet, solche Testwerte für die atrioventrikuläre Verzögerungszeit und die interventrikuläre Verzögerungszeit zu wählen, für die der höchste HDB gespeichert ist. Dies soll vorzugsweise dann geschehen, wenn keine leeren oder veralteten Matrixfelder mehr vorliegen.

Diese Ausgestaltungen des Schrittmacherzeitgebers haben zur Folge, dass nach möglichst kurzer Zeit möglichst alle geeigneten Werte der atrioventrikulären Verzögerungszeit und der interventrikulären Verzögerungszeit gespeichert sind, die den mit den jeweiligen Werten für die atrioventrikuläre Verzögerungszeit und die interventrikuläre Verzögerungszeit verbundenen HDB charakterisieren. Die letztgenannte Ausführungsvariante trägt dazu bei, dass die jeweils gespeicherten, den HDB charakterisierenden Werte möglichst aktuell sind.

Gemäß der Erfindung ist der Schrittmacherzeitgeber dazu ausgebildet, von einem durch aktuelle Werte der atrioventrikulären Verzögerungszeit (AVD_{Akt}) und der interventrikulären Verzögerungszeit (VVD_{Akt}) definierten Arbeitspunkt ausgehende Testwerte für die atrioventrikuläre Verzögerungszeit und die interventrikuläre Verzögerungszeit zu bestimmen, die in einer vom Arbeitspunkt ausgehenden Messrichtung liegen, wobei der Schrittmacherzeitgeber weiter dazu ausgebildet ist, die Messrichtung durch Auswertung gespeicherter Werte für die atrioventrikuläre und die interventrikuläre Verzögerungszeit sowie zugehörige, den jeweiligen HDB charakterisierende Werte zu bestimmen. Auf diese Weise ist der Schrittmacherzeitgeber in der Lage, bevorzugt solche Testwerte auszuwählen, die aufgrund bisher erfasster Werte zum HDB einen größeren HDB erwarten lassen. Der Schrittmacherzeitgeber ist auf diese Weise in der Lage, möglichst schnell Maxima des HDB zu finden.

Um die optimale Richtung auch bei kleinen Veränderungen, die sich einer direkten Auswertung entziehen, bestimmen zu können ist auch die Anwendung der Trendanalyse über mehrere in unmittelbarer zeitlichen und räumlichen Nachbarschaft ermittelten Ergebnisse anzuwenden.

In jedem Fall ist ein Schrittmacherzeitgeber bevorzugt, der immer dann die Testwerte für die atrioventrikuläre Verzögerungszeit und die interventrikuläre Verzögerungszeit zu neuen Arbeitswerten der atrioventrikulären Verzögerungszeit und der interventrikulären Verzögerungszeit für die weitere reguläre Stimulation verwendet, wenn ein mit jeweils aktuell getesteten Testwerten für die atrioventrikuläre Verzögerungszeit (AVD_{Test}) und die interventrikuläre Verzögerungszeit (VVD_{Test}) verbundener HDB höher als bei den bisherigen Arbeitswerten ist. Vorzugsweise wird noch geprüft, ob der Unterschied im HDB ein vorgegebenes Mindestmaß überschreitet.

Gemäß einer weiteren bevorzugten Ausführungsvariante ist der Schrittmacherzeitgeber dazu ausgebildet, Testwerte für die atrioventrikuläre Verzögerungszeit und die interventrikuläre Verzögerungszeit zu verwerfen, sofern diese oder der mit ihnen verbundene, den HDB charakterisierende Wert vorgegebene Qualitätsbedingungen nicht erfüllen. Auf diese Weise ist der Schrittmacherzeitgeber in der Lage, ungeeignete Werte für die atrioventrikuläre und/oder die interventrikuläre Verzögerungszeit auszuschließen. Falls individuelle, Patienten abhängige Einschränkungen bezüglich des zumutbaren und sicheren Bereiches der AVD/VVD Parameter bekannt sind ist auch die Einschränkung des AVD/VVD Bereiches möglich.

Für den Fall, dass die Qualitätsbedingungen für mindestens eine bestimmte Zeit oder Zyklenzahl nicht erfüllt sind, ist der Schrittmacherzeitgeber vorzugsweise ausgebildet, automatisch auf eine alternative Methode zur Bestimmung von AVD und VVD umzuschalten, z. B. auf eine Methode, die durch Langzeitmittelung das Rauschen reduziert, oder sogar auf eine Methode, die AVD und VVD mit Hilfe fester programmierter Parameter in Abhängigkeit von HR und dem atrialen Ereignistyp, bestimmt.

Gemäß weiterer, bevorzugter Ausführungsvarianten ist der Schrittmacherzeitgeber ausgebildet, intrinsische atrioventrikuläre Überleitungen zuzulassen und durch entsprechende Variationen der Arbeitswerte für AVD und VVD zu fördern.

Außerdem ist der Schrittmacherzeitgeber vorzugsweise ausgebildet, in wiederkehrenden Zeitintervallen zufällig bestimmte Werte für AVD_{Test} und VVD_{Test} unabhängig von einer aktuellen Messrichtung MR auszuwählen und anzuwenden, um zu vermeiden, dass der Schrittmacherzeitgeber dauerhaft an Stimulationsparametern, insb. AVD- und VVD-Werten festhält, die lediglich zu einem lokalen, nicht aber zu einem globalen Maximum des HDB führen.

Weitere vorteilhafte Ausgestaltungen der Erfindung ergeben sich aus der nachfolgenden Beschreibung eines Ausführungsbeispiels.

Die Erfindung soll nun anhand eines Ausführungsbeispiels mit Bezug auf die Figuren näher erläutert werden. Von den Figuren zeigen:
- Figur 1:: Eine erfindungsgemäße Vorrichtung in Form eines Herzschrittmachers samt daran angeschlossener Elektroden. Die Lage der Elektroden in Bezug auf ein menschliches Herz ist skizzenhaft dargestellt. In Figur 1 unten links ist der Zeitverlauf des zur Impedanzmessung eingespeisten Stroms eine skiz- zenhaft dargestellt;
- Figur 2:: ein Blockschaltbild wesentlicher Komponenten des Herzschrittmachers aus Figur 1;
- Figur 3:: ein Blockschaltbild wesentlicher Komponenten des Herzschrittmachers aus Figur 1 in einer zu Figur 2 alternativen Ausführungsvariante;
- Figur 4:: ein beispielhaftes Flächendiagramm, das einen HDB für verschiedene Werte von AVD und VVD darstellt.
- Figur 5:: ein allgemeines Ablaufdiagramm zur Beschreibung der Funktionsweise der Schrittmachersteuerung und ihres Schrittmacherzeitgebers im Falle des Tes- tens von Werten von AVD und VVD sowie der Speicherung des jeweiligen Testergebnisses;
- Figur 6:: ein beispielhaftes Ablaufdiagramm zur Beschreibung der Funktionsweise der Schrittmachersteuerung und ihres Schrittmacherzeitgebers im Falle des Testens von Werten für die atrioventrikuläre Verzögerungszeit AVD;
- Figur 7:: ein beispielhaftes Ablaufdiagramm zur Beschreibung der Funktionsweise der Schrittmachersteuerung und ihres Schrittmacherzeitgebers im Falle des Testens von Werten für die interventrikuläre Verzögerungszeit VVD;
- Figur 8:: ein beispielhaftes Ablaufdiagramm zur Beschreibung der Funktionsweise der Schrittmachersteuerung und ihres Schrittmacherzeitgebers bei Bestim- men von Testwerten für AVD und VVD sowie der Speicherung der Messer- gebnisse für den jeweils zugehörigen HDB; und
- Figur 9:: eine alternative Darstellung zur Illustration der Funktionsweise der Schritt- machersteuerung und ihres Schrittmacherzeitgebers im Falle des Testens von Werten von AVD und VVD sowie der Speicherung des jeweiligen Testergebnisses.

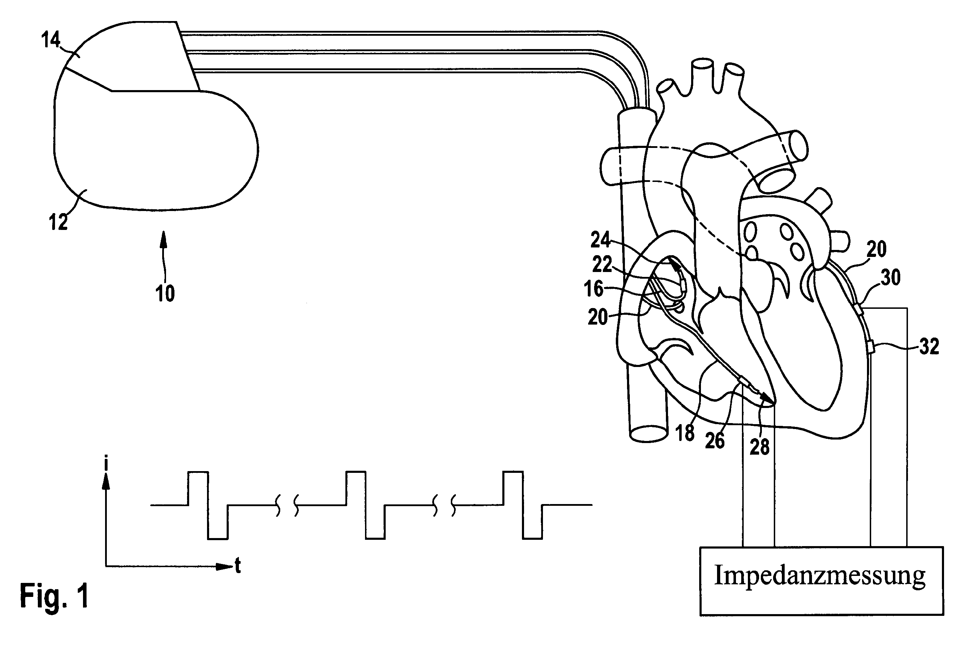

In Figur 1 ist ein biventrikulärer Herzstimulator 10 dargestellt, der über Elektrodenleitungen mit Wahrnehmungs- und Stimulationselektroden im rechten Atrium, im rechten Ventrikel und nahe dem linken Ventrikel eines Herzens verbunden ist.

Der Herzstimulator 10 besitzt ein hermetisch dichtes Metallgehäuse 12 und einen Anschlusskopf (Header) 14 aus transparentem Kunststoff, der mehrere Buchsen zum Anschluss der Elektrodenleitungen aufweist. Die Elektrodenleitungsanschlüsse im Header 14 sind elektrisch mit einer Steuerschaltung im Inneren des Gehäuses des Herzschrittmachers 10 verbunden.

An die Elektrodenleitungsanschlüsse sind insgesamt drei Elektrodenleitungen angeschlossen, nämlich eine rechtsatriale Elektrodenleitung 16, eine rechtsventrikuläre Elektrodenleitung 18 und eine linksventrikuläre Elektrodenleitung 20.

Die rechtsatriale Elektrodenleitung 16 trägt eine rechtsatriale Ringelektrode 22 und eine rechtsatriale Spitzenelektrode (Tipelektrode) 24. Die rechtsventrikuläre Elektrodenleitung 18 trägt eine rechtsventrikuläre Ringelektrode 26 und eine rechtsventrikuläre Spitzenelektrode (Tipelektrode) 28. Die linksventrikuläre Elektrodenleitung 20 ist über das rechte Atrium des in Figur 1 schematisch dargestellten Herzens und den Coronar Sinus des Herzens bis in die Peripherie des linken Ventrikels geführt. Die linksventrikuläre Elektrodenleitung 20 trägt eine linksventrikuläre Ringelektrode 30 und eine linksventrikuläre Spitzenelektrode 32.

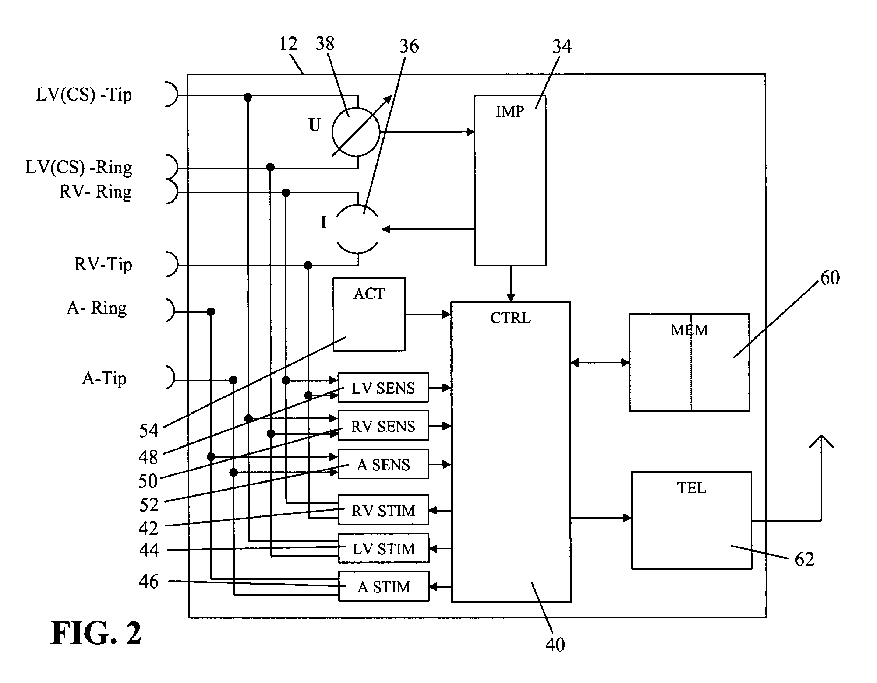

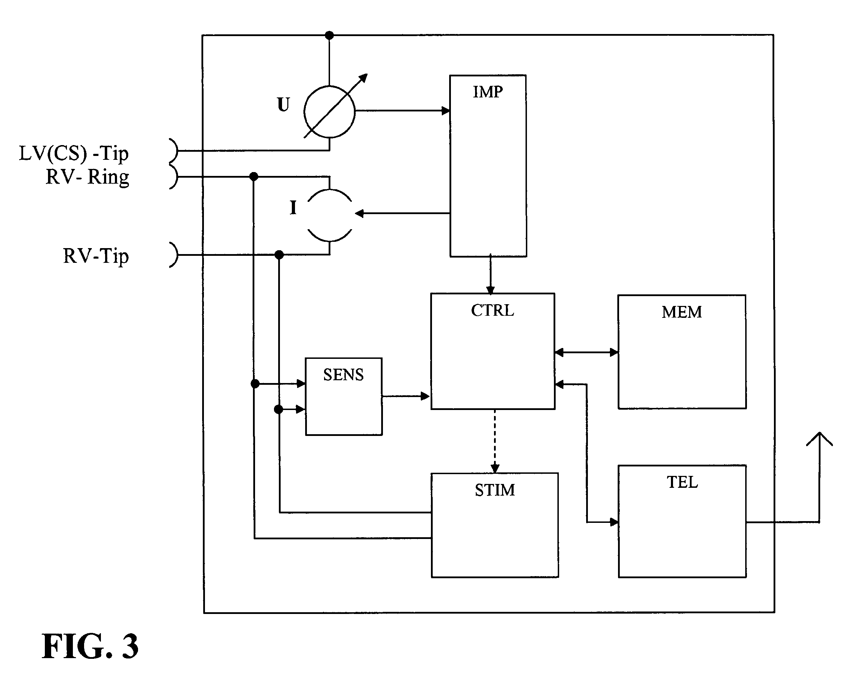

Zum Bestimmen eines HDB besitzt der Herzstimulator 10 eine Impedanzmesseinheit (siehe Figuren 2 und 3), die in der in Figur 2 dargestellten Ausführungsvariante zum Zwecke der Impedanzmessung mit der rechtsventrikulären Ringelektrode 26 und der rechtsventrikulären Spitzenelektrode 28 sowie der linksventrikulären Ringelektrode 30 und der linksventrikulären Spitzenelektrode 32 verbunden ist.

Die Abgabe eines biphasischen gepulsten Messstroms, wie er in Figur 1 unten links skizzenhaft dargestellt ist, erfolgt über die rechtsventrikuläre Ringelektrode 26 und die rechtsventrikuläre Spitzenelektrode 28, also im rechten Ventrikel. Die Messung der durch den Strom hervorgerufenen Spannung erfolgt über die linksventrikuläre Ringelektrode 30 und die linksventrikuläre Spitzenelektrode 32. Die Rolle der Elektroden zur Stromeinspeisung und Spannungsmessung kann auch vertauscht sein.

Wie der Darstellung unten links in Figur 1 zu entnehmen ist, wird der Strom zur Impedanzmessung in biphasischen Impulsen abgegeben, wobei zwei gegenphasige Konstantstromimpulse unmittelbar aufeinander folgen und jeweils ein Impulspaket bilden. Die einzelnen Impulspakete haben einen zeitlichen Abstand voneinander, der wesentlich größer ist als die Dauer des jeweiligen Impulspaketes. Die beiden Gleichstromimpulse innerhalb des Impulspaketes besitzen jeweils die gleiche Amplitude, jedoch mit unterschiedlicher Polung und sind jeweils gleich lang. Typische Werte für den Maximalstrom der Gleichstromimpulse liegen zwischen 50 Mikroampere und 600 Mikroampere. Eine typische Impulsdauer eines einzelnen Strompulses kann 15 Mikrosekunden betragen. Die Impulspakete werden in regelmäßigen Anständen erzeugt, typischerweise mit Wiederholungsfrequenzen zwischen 30 und 300 Hz.

Anders als in Figur 1 unten links dargestellt können die beiden Gleichstromimpulse eines Impulspaketes auch mit einem zeitlichen Abstand aufeinander folgen, der der Dauer eines Gleichstromimpulses entspricht. Zwischen zwei gegenphasigen Gleichstromimpulsen eines Impulspaketes ergibt sich dann jeweils eine Lücke von der Dauer eines Gleichstromimpulses, während der kein Gleichstrom abgegeben wird. Eine weitere Variante besteht darin, dass die Impulspakete phasenalternierend abgegeben werden, das heißt, dass streng abwechselnd ein Impulspaket beispielsweise mit einem negativen Gleichstromimpuls beginnt und mit einem positiven Gleichstromimpuls endet und das darauf folgende Impulspaket mit einem positiven Gleichstromimpuls beginnt und mit einem negativen Gleichstromimpuls endet und so fort. Durch diese phasenalternierende Abgabe von Impulspaketen wird eine Aufladung der Elektrodengrenzflächen verhindert und es werden Artefakte vermieden.

Figuren 2 und 3 zeigen jeweils ein Blockschaltbild mit den in Bezug auf die hier betroffene Erfindung wesentlichen Komponenten einer Schaltung im Inneren des Gehäuses 12 des Herzstimulators 10.

Diese Bestandteile sind eine Impedanzmesseinheit IMP, die einerseits mit einem Konstantstromgenerator I verbunden ist, der einen gepulsten biphasischen Konstantstrom erzeugt und über einen Anschluss RV-Ring für die rechtsventrikuläre Ringelektrode 26 und einen Anschluss RV-Tip für die rechtsventrikuläre Spitzenelektrode 28 abgibt. Außerdem ist die Impedanzmesseinheit mit einer Spannungsmesseinheit U verbunden, die ihrerseits mit zwei Anschlüssen verbunden ist, über die die jeweilige Spannung erfasst wird, die der zu Impedanzmesszwecken abgegebene Konstantstrom hervorruft. Die Spannungsmesseinheit U ist im Falle der bevorzugten Ausführungsvariante gemäß Figur 2 mit einem Anschluss LV-Ring für die linksventrikuläre Ringelektrode 30 sowie mit einem Anschluss LV-Tip für die linksventrikuläre Spitzenelektrode 32 verbunden. In einer alternativen Ausführungsvariante (siehe Figur 3) ist die Spannungsmesseinheit U einerseits mit einem Anschluss LV-Tip für die linksventrikuläre Spitzenelektrode 32 verbunden und andererseits mit dem elektrisch leitenden Gehäuse 12 des Herzschrittmachers 10.

In dem Gehäuse 12 des Herzstimulators 10 ist außerdem eine Schrittmachersteuerung CTRL40 enthalten, die einen Schrittmacherzeitgeber enthält (nicht dargestellt). Die Schrittmachersteuerung 40 ist mit einer rechtsventrikulären Stimulationseinheit 42, einer linksventrikulären Stimulationseinheit 44 und einer atrialen Stimulationseinheit 46 verbunden. Die rechtsventrikuläre Stimulationseinheit 42 ist ihrerseits zum einen mit einem elektrischen Anschluss für die rechtsventrikuläre Ringelektrode RV-Ring verbunden und zum anderen mit einem elektrischen Anschluss für die rechtsventrikuläre Tipelektrode RV-Tip. Entsprechend ist die linksventrikuläre Stimulationseinheit 44 mit einem elektrischen Anschluss für die linksventrikuläre Ringelektrode RV-Ring und einem elektrischen Anschluss für die linksventrikuläre Tipelektrode RV-Tip verbunden. Die atriale Stimulationseinheit 46 ist analog mit einem elektrischen Anschluss für die atriale Ringelektrode A-Ring und einem weiteren elektrischen Anschluss für die atriale Tipelektrode A-Tip verbunden.

Die Stimulationseinheiten 42, 44 und 46 sind jeweils dazu ausgebildet, auf ein Triggersignal der Schrittmachersteuerung 40 hin einen Stimulationsimpuls zu erzeugen und diesen über die elektrischen Anschlüsse an die daran angeschlossenen Elektroden und - im implantierten Zustand - an das Herzgewebe der jeweiligen Herzkammer abzugeben. Der Stimulationsimpuls ist dabei jeweils so bemessen, dass er zu einer stimulierten Kontraktion des Herzgewebes führt, wie dies dem hier einschlägigen Fachmann bekannt ist.

Darüber hinaus ist die Schrittmachersteuerung 40 mit einer linksventrikulären Wahrnehmungseinheit LV-Sense 48, einer rechtsventrikulären Wahrnehmungseinheit RV-Sense 50 und einer atrialen Wahrnehmungseinheit A-Sense 52 verbunden. Wie die entsprechenden Stimulationseinheiten sind auch die Wahrnehmungseinheiten 48, 50 und 52 mit den elektrischen Anschlüssen für die entsprechenden Elektroden verbunden. Die Wahrnehmungseinheiten 48, 50 und 52 dienen in bekannter Manier dazu, über die an die angeschlossenen Elektroden aufgenommene elektrische Potenziale zu verstärken und das sich ergebende Signal hinsichtlich solcher Signalmerkmale auszuwerten, die eine natürliche Kontraktion der jeweiligen Herzkammer anzeigen. Im einfachsten Fall detektiert die jeweilige Wahrnehmungseinheit 48, 50 oder 52 eine Schwellwertüberschreitung des jeweils auszuwertenden Signals und generiert daraufhin ein Sense-Signal, das ein wahrgenommenes Ereignis (Sense-Ereignis) charakterisiert, welches als Hinweis auf eine natürliche Kontraktion der entsprechenden Herzkammer ausgewertet wird. Diese Signale gibt die jeweilige Wahrnehmungseinheit 48, 50 oder 52 an die Schrittmachersteuerung 40 ab.

Die Schrittmachersteuerung 40 reagiert auf ein jeweiliges Sense-Signal, indem sie beispielsweise in bekannter Manier die Abgabe eines nächsten, bereits vorgesehenen Stimulationsimpulses unterdrückt. Gleichzeitig kann ein Sense-Signal ein Intervall auslösen, welches den Zeitpunkt der Abgabe eines nächsten vorgesehenen Stimulationsimpulses bestimmt. Auch dies ist grundsätzlich bekannt.

Ein Aktivitätssensor ACT 54 ist ebenfalls mit der Schrittmachersteuerung 40 verbunden und erlaubt es der Schrittmachersteuerung 40 bzw. deren Schrittmacherzeitgeber, eine jeweilige Stimulationsrate an den hämodynamischen Bedarf eines Patienten anzupassen, so dass der Herzstimulator 10 ratenadaptiv ist. Der Aktivitätssensor ACT 54 kann beispielsweise ein Akzelerometer, ein Impedanzsensor (z. B. Minutenvolumen-Impedanz-Sensor, oder "Closed-Loop"_Impedanzsensor), oder ein anderer physiologischer Sensor für die momentane Belastung des Patienten sein.

Wie eingangs erwähnt ist es eine wesentliche Aufgabe des Schrittmacherzeitgebers und damit der Schrittmachersteuerung 40, geeignete Zeitpunkte für die Abgabe eines jeweiligen Stimulationsimpulses an die jeweilige Kammer eines Herzens so zu bestimmen, dass die Stimulation des Herzens zu einem möglichst großen HDB führt.

Neben der Aufgabe, eine möglichst von der körperlichen Aktivität des Patienten abhängige generelle Stimulationsrate (der Rate, mit der gleichartige Herzaktionen, beispielsweise ventrikuläre Kontraktionen, aufeinander folgen) zu bestimmen, hat der Schrittmacherzeitgeber die Aufgabe, geeignete Intervalle zu bestimmen, die die Zeitpunkte der Stimulationsimpulsabgabe an die einzelnen Kammern innerhalb eines Herzzyklus definieren. Diese Intervalle sind insbesondere ein atrioventrikuläres Verzögerungsintervall AVD, das mit einem natürlichen (wahrgenommenen) atrialen Ereignis oder einem atrialen Stimulus gestartet wird und zu dessen Ende ein rechtsventrikulärer Stimulationsimpuls ausgelöst wird, sofern er nicht infolge eines zuvor erfassten, natürlichen rechtsventrikulären Ereignisses unterdrückt wird, sowie ein interventrikuläres Verzögerungsintervall VVD, das den zeitlichen Abstand zwischen einem vorgesehenen rechtsventrikulären Stimulus und einem vorgesehenen linksventrikulären Stimulus bestimmt. Typischerweise wird der Zeitpunkt der vorgesehenen Abgabe eines linksventrikulären Stimulus ausgehend von dem Zeitpunkt berechnet, zu dem ein rechtsventrikulärer Stimulus vorgesehen ist. Dabei kann die interventrikuläre Verzögerungszeit VVD sowohl positiv als auch negativ sein, d. h. der vorgesehene linksventrikuläre Stimulus kann vor oder nach dem vorgesehenen rechtsventrikulären Stimulus eines Zeitzyklus liegen.

Der Herzstimulator 10 ist dazu ausgebildet, möglichst schnell geeignete, an eine jeweilige Situation und einen jeweiligen Patienten angepasste Werte für die atrioventrikuläre Verzögerungszeit AVD und die interventrikuläre Verzögerungszeit VVD zu bestimmen. Hierzu testet die Schrittmachersteuerung 40 zu regelmäßig oder unregelmäßig wiederkehrenden Zeitpunkten jeweils Paare von Werten für die atrioventrikuläre Verzögerungszeit AVD und die interventrikuläre Verzögerungszeit VVD.

Der mit diesen jeweils zu testenden Paaren von Werten AVD_{Test} und WD_{Test} für die atrioventrikuläre Verzögerungszeit bzw. die interventrikuläre Verzögerungszeit verbundene HDB ermittelt die Schrittmachersteuerung 40 mit Hilfe der Impedanzmesseinheit 34. Der mittels der Impedanzmesseinheit 34 ermittelte Verlauf der intrakardialen Impedanz ist ein Indiz für einen jeweils erzielten HDB.

Alternativ kann dieser auch auf dem Wege der Auswertung eines Druck-Volumen-Diagramms bestimmt werden, wie es in der parallel anhängigen Anmeldung [DE 10 2009 002 397.6] im Einzelnen beschrieben ist.

Der zu einem jeweiligen Wertepaar von AVD_{Test} und VVD_{Test} ermittelte HDB wird nach Art einer Matrix den beiden Werten für AVD_{Test} und VVD_{Test} zugeordnet gespeichert. Hierzu ist die Schrittmachersteuerung 40 mit einem Speicher MEM 60 verbunden. Dies erlaubt es der Schrittmachersteuerung und insbesondere dem Schrittmacherzeitgeber, zu einem späteren Zeitpunkt auf diese Werte zurückzugreifen, um für einen jeweiligen aktuellen Bedarf möglichst optimale Werte für AVD und VVD für die Zeitsteuerung zu nutzen.

Ein Beispiel für eine derart aufgenommene Werte-Matrix ist in Figur 1 abgebildet. In der Figur ist der HDB für alle möglichen Kombinationen aus AVD und VVD in einem schematischen Flächendiagramm aufgetragen. Es ist sinnvoll, mehrere solcher Flächendiagramme in Abhängigkeit von Herzrate, atrialen Ereignistyp und Aktivitätsgrad zu erstellen, da sich die entsprechenden Optima bei Belastung verschieben.

Figur 1 zeigt beispielhaft eine aktuelle Einstellung für die atrioventrikuläre Verzögerungszeit AVD_{Akt} und die interventrikuläre Verzögerungszeit VVD_{Akt} mit AVD_{Akt} = 70 ms und VVD_{Akt} = -10 ms. Dieser Arbeitspunkt ist in Figur 1 durch einen Kreis dargestellt. Pfeile markieren verschiedene Änderungsrichtungen für AVD und VVD.

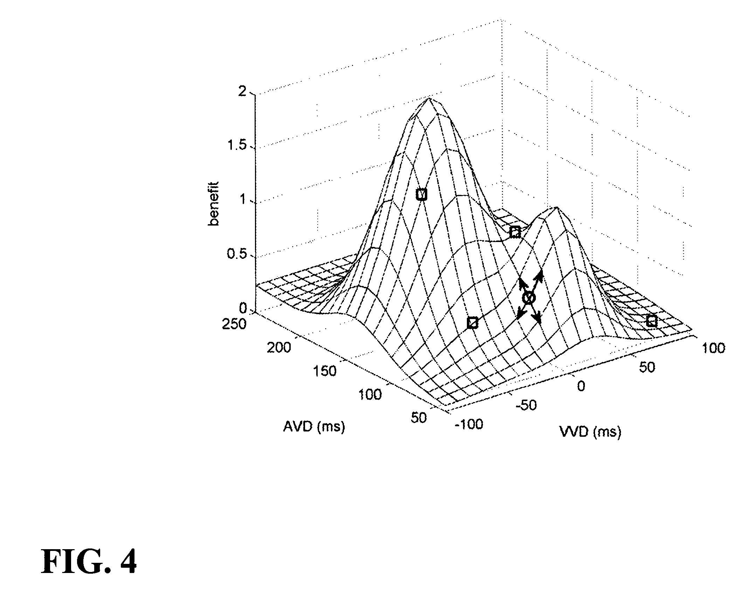

Figur 4 zeigt auch beispielhaft, dass die den HDB repräsentierende Fläche drei Maxima besitzen kann, nämlich zwei lokale Maxima, bei AVD = 120 ms und VVD = 0 ms und bei AVD 80 ms und VVD = -10 sowie ein globales Maximum bei AVD = 90 ms und VVD = 40 ms.

Um Werte aufzunehmen, die sich in Form einer Matrix, wie der in Figur 4 dargestellten, wiedergeben lassen, ist der Schrittmacher dazu ausgebildet, zu bestimmten Zeitpunkten T_{VAR} Variationen der atrioventrikulären Verzögerungszeit und der interventrikulären Verzögerungszeit vorzunehmen und von den aktuellen Werten dieser Verzögerungszeiten AVD_{Akt} und VVD_{Akt} abweichende Testwerte dieser Verzögerungszeiten AVD_{Test} und VVD_{Test} anzuwenden. Diese Testwerte der atrioventrikulären Verzögerungszeit AVD_{Test} und der interventrikulären Verzögerungszeit VVD_{Test} führen zu einem HDB, der, wie zuvor beschrieben, durch Impedanzmessung oder durch Aufnahme und Auswertung eines Druck-Volumen-Diagramms, oder eines anderen hämodynamischen Sensors, quantifiziert wird. Der so gewonnene, den jeweiligen HDB charakterisierende Wert wird dem jeweils angewandten Wertepaar AVD_{Test} und VVD_{Test} zugeordnet gespeichert.

Bei der Auswahl der jeweils zu testenden Werte für die atrioventrikuläre Verzögerungszeit und die interventrikuläre Verzögerungszeit fragt der Schrittmacherzeitgeber nach einer vorgebenden Anzahl von Testmesszyklen die bereits gespeicherten Werte ab, um zu überprüfen, ob es noch geeignete Wertepaare von AVD und VVD gibt, die bisher nicht getestet wurden und für die daher kein den HDB charakterisierender Wert gespeichert ist. In diesem Falle testet der Schrittmacherzeitgeber solche bisher nicht getesteten Arbeitspunkte wenn sich keine sonstigen Einträge in der Matrix befinden, die außerhalb der unmittelbaren Umgebung um den Arbeitspunkt ein globales Optimum vermuten lassen.

Gemäß einer bevorzugten Ausführungsvariante wird zu jedem Wertetripel AVD, VVD und HDB auch das Datum gespeichert, zu dem das jeweilige Wertetripel gebildet wurde. Vor dem Testen weiterer Werte von AVD und VVD fragt der Schrittmacherzeitgeber den Speicher hinsichtlich des Alters der bereits getesteten Wertepaare ab und testet bevorzugt solche Wertepaare, die vergleichsweise alt sind.

Weiterhin ist der Schrittmacherzeitgeber dazu ausgebildet, solche Testwerte für die atrioventrikuläre Verzögerungszeit und die interventrikuläre Verzögerungszeit zu wählen, für die der höchste HDB gespeichert ist. Dies soll vorzugsweise dann geschehen, wenn keine leeren oder veralteten Matrixfelder mehr vorliegen.

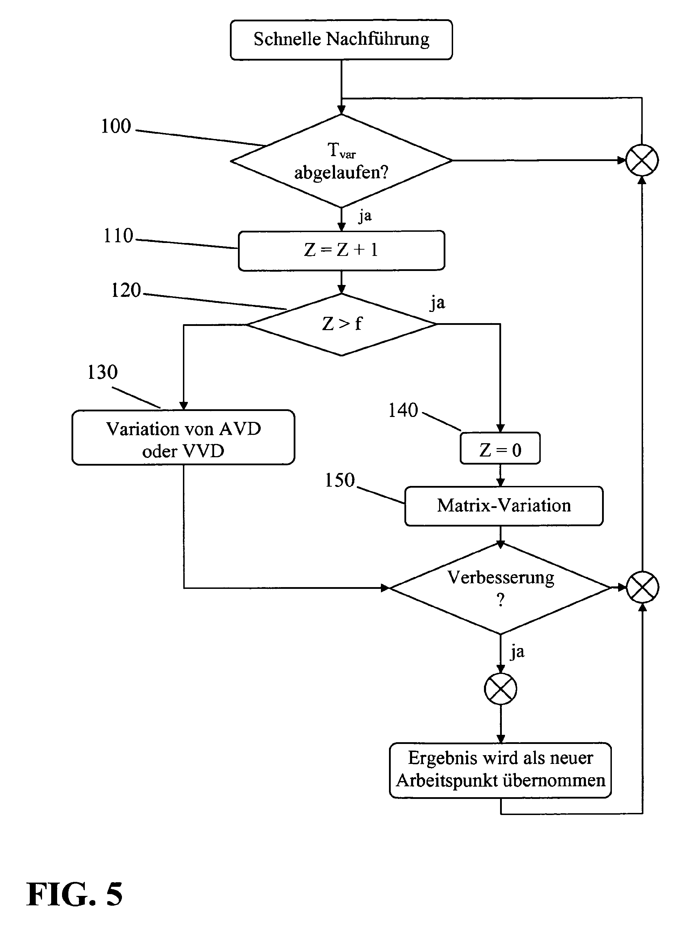

Diese gezielte Vorgehensweise des Schrittmacherzeitgebers ist in den Ablaufdiagrammen in den Figuren 5 bis 9 detaillierter dargestellt.

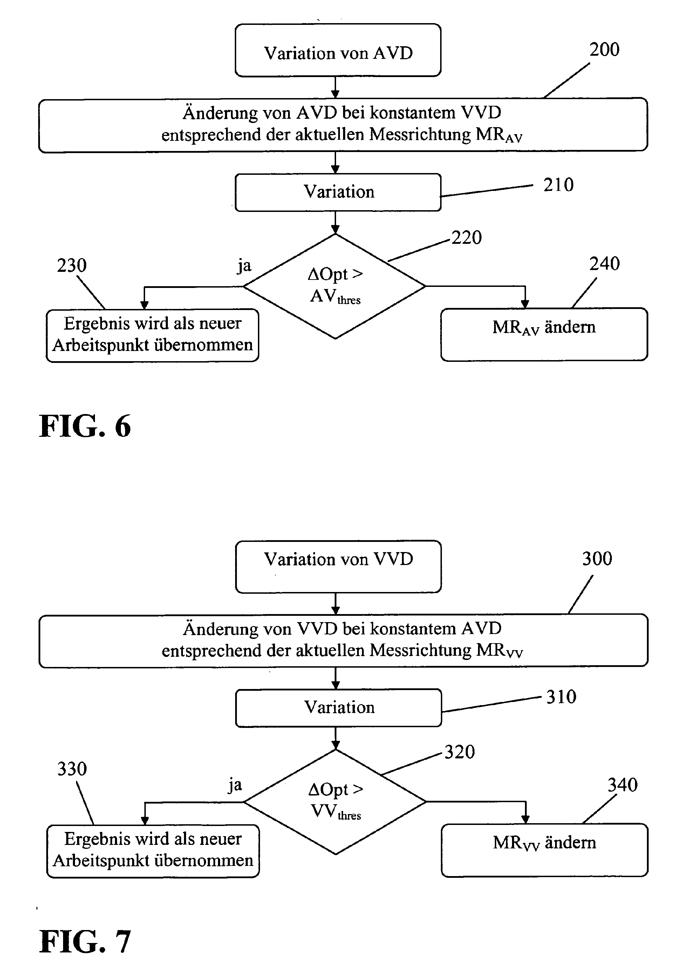

Figur 5 zeigt, dass Variationen der Werte für AVD und VVD in Zeitintervallen von T_{VAR} wiederholt werden (100). Ist das jeweilige Zeitintervall T_{VAR} abgelaufen, wird ein Zähler Z gestartet (110) und eine Variation von AVD und/oder VVD (130) über eine Anzahl von f Herzzyklen vorgenommen (120). Die Variation der atrioventrikulären Verzögerungszeit ist dabei in Figur 6 dargestellt, während die Variation der interventrikulären Verzögerungszeit in Figur 7 dargestellt ist.

Für die jeweilige Variation erfolgt die Änderung von AVD oder VVD gegenüber einem jeweiligen aktuellen Arbeitspunkt in Richtung einer aktuellen Messrichtung MR_{AV} bzw. MR_{VV} (siehe 200 bzw. 300). Der Schrittmacherzeitgeber wendet die zu testenden Verzögerungszeiten AVD_{Test} und VVD_{Test} an; siehe Figur 6 bei 210 und Figur 7 bei 310. In beiden Fällen wird nach Anwendung der zu testenden Werte der atrioventrikulären Verzögerungszeit und der interventrikulären Verzögerungszeit AVD_{Test} und VVD_{Test} geprüft, ob eine Veränderung des HDB, ΔOpt (das ist der Unterschied im hämodynamischen Zustand zwischen den Testwerten AVDₜₑₛₜ&VVDₜₑₛₜ und den bisherigen Arbeitswerten für AV-Dₐₖₜ&VVDₐₖₜ), größer ist als ein jeweiliger Schwellwert THRES_{AV} oder THRES_{VV} für eine hämodynamische Verbesserung in Folge der AVD Variation bzw. der VVD Variation (220 bzw. 320). Nur wenn die Veränderung des Optimierungskriteriums ΔOpt größer ist als der jeweils vorgegebene Schwellwert THRES_{AV} bzw. THRES_{VV}, wird das aktuell getestete Paar aus AVD und VVD als neuer aktueller Arbeitspunkt mit den Werten AVDₙₑᵤ = AVDTest und VVDₙₑᵤ = VVD_{Test} übernommen; siehe 230 bzw. 330. Überschreitet der die Änderung des Optimierungskriteriums charakterisierende Wert ΔOpt den jeweiligen Schwellwert THRES_{AV} bzw. THRES_{VV} nicht, ändert der Schrittmacherzeitgeber die Messrichtung MR_{AV} bzw. MR_{VV} um eventuell besser geeignete Testwerte AVD_{Test} und VVD_{Test} und damit ggf. neue Arbeitswerte AVDₙₑᵤ und VVDₙₑᵤ zu finden (siehe 240 bzw. 340).

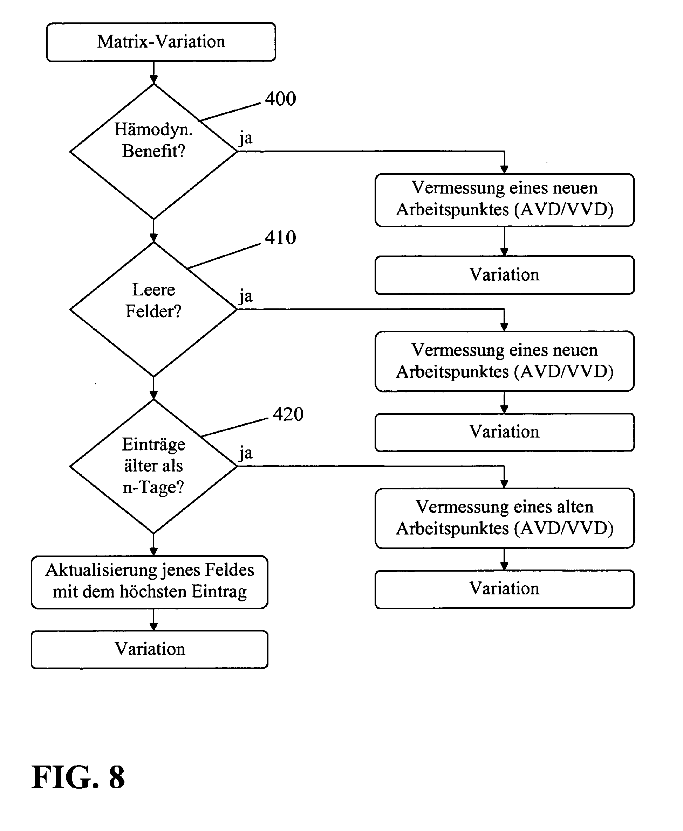

Wenn der Schrittmacherzeitgeber auf diese Weise die Variation der atrioventrikulären Verzögerungszeit und der interventrikulären Verzögerungszeit durchgeführt und die durch den Zähler Z definierte Anzahl von zu testenden Werten der atrioventrikulären Verzögerungszeit und der interventrikulären Verzögerungszeit getestet und gegebenenfalls einen neuen Arbeitspunkt gefunden hat, setzt der Schrittmacherzeitgeber den Zähler Z zurück auf 0 (140) und führt eine Matrixvariation (150) durch, wie sie in Figur 8 abgebildet ist. Wie Figur 8 zu entnehmen ist, wählt der Schrittmacherzeitgeber bevorzugt solche Werte AVD_{Test} und VVD_{Test} aus, für die noch kein den HDB beschreibender Wert erfasst ist (siehe 400). Sind bereits sämtliche möglichen Arbeitspunkte vermessen, prüft der Schrittmacherzeitgeber, ob die Vermessung einiger Arbeitspunkte schon mehr als n Tage zurückliegt (siehe 410) und testet dann die den jeweiligen Arbeitspunkt zugeordneten Werte der AVD und der VVD. Für den Fall, dass sich in der Matrix ein HDB befindet der bessere Ergebnisse verspricht als dies für den aktuellen Arbeitspunkt zutrifft, so wird dieses Wertepaar neu vermessen und gegebenenfalls als neuer Arbeitspunkt übernommen. Voraussetzung dafür ist allerdings, dass sich diese AVD/VVD Kombination nicht in direkter Nachbarschaft des aktuellen Arbeitspunktes befindet; siehe 420.

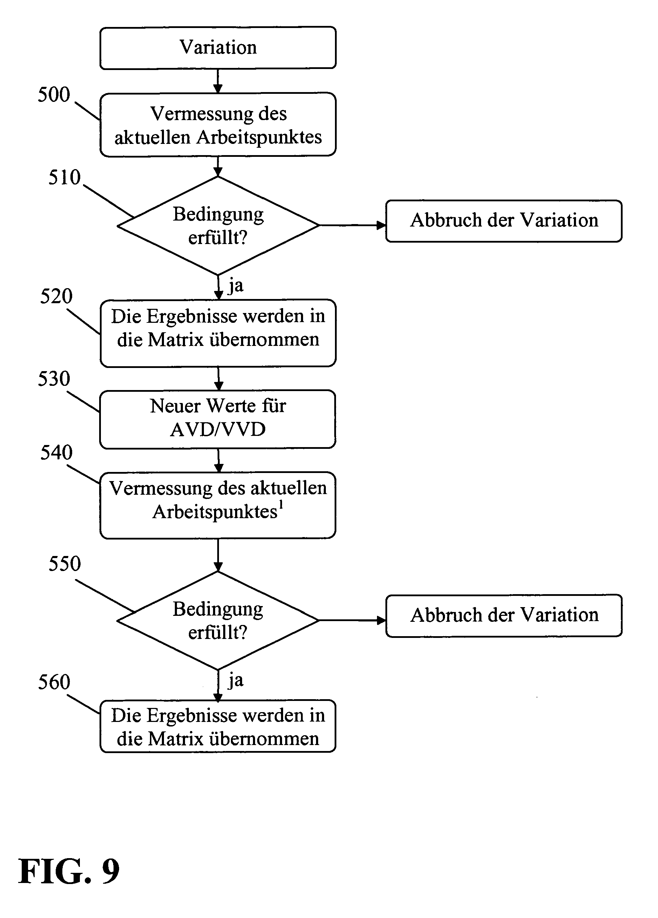

Beim Testen verschiedener Arbeitspunkte berücksichtigt der Schrittmacherzeitgeber vorgegebene Qualitäts- und Abbruchkriterien. Dies ist in Figur 9 dargestellt. Die Vermessung eines aktuellen Arbeitspunktes (500) erfolgt dadurch, dass ein hämodynamisch relevantes Sensorsignal (beispielsweise die Impedanz) für eine ausreichende Anzahl von aufeinander folgenden Herzzyklen aufgezeichnet wird um eine ausreichende Rauschunterdrückung gewährleisten zu können. Hierbei wird überprüft, ob das jeweils über 2ⁿ Herzzyklen gemittelte hämodynamische Signal, beispielsweise die Impedanz, vorgegebene Qualitätsbedingungen erfüllt; siehe 510. Ist dies nicht der Fall, erfolgt der Abbruch des Testes mit jenen Testwerten AVD_{Test} und VVD_{Test}. Nur wenn die Qualitätsbedingungen erfüllt sind, werden die Ergebnisse als Wertetripel gespeichert, d. h. das jeweilige Sensorausgangssignal (beispielsweise die Impedanz) in die von AVD und VVD definierte Matrix übernommen (520). Anschließend werden neue Werte für AVD und VVD getestet (530). Diese bilden einen neuen aktuellen Arbeitspunkt, der genau, wie zuvor geschildert, vermessen wird (540). Auch in diesem Falle wird wieder das Erfüllen der Qualitätsbedingungen geprüft (550). Nur wenn die Qualitätsbedingungen erfüllt sind, werden auch die Werte für die getesteten Verzögerungszeiten AVD_{Test} und VVD_{Test} gespeichert, d. h. in die Matrix übernommen (560). Ansonsten erfolgt ein Abbruch des Tests.

Die Qualitätsbedingungen können durch bestimmte Frequenzkriterien oder eine vorgegebene Kontraktionsdynamik, oder das Rauschen oder Störungen auf dem Sensorsignal, definiert sein.

Weiterhin kann der Schrittmacherzeitgeber ausgebildet sein, das Messrauschen des oder der Sensoren, die zur Bestimmung des HDB verwendet werden, zu bestimmen und in weiterer Folge zur Parameteroptimierung zu verwenden. Also wird z. B. bei günstigen Rauschverhältnissen eine schnellere Reaktionszeit durch eine geringere Anzahl von gemittelten Zyklen möglich, oder man reduziert die Rauschsensitivität durch größere Schrittweiten, oder längere Mittelungen. Auch ist die Umschaltung zu einer anderen Optimierungsmethode in Abhängigkeit vom Rauschen sinnvoll.

Der Schrittmacherzeitgeber ist somit ausgebildet, einen Wert für AVD und der VVD laufend, mit einer Antwortzeit von 1- 10 Minuten, den sich ändernden Bedingungen anzupassen.

Dies geschieht wie folgt:

Ausgehend von einer aktuellen Einstellung der atrioventrikulären Verzögerungszeit mit einem Wert AVDₐₖₜ und der interventrikulären Verzögerungszeit mit einem Wert VVDₐₖₜ stellt der Schrittmacherzeitgeber jeweils für wenige Herzschläge "benachbarte" Werte als zu testende Werte (Testwerte) AVD_{Test} und VVD_{Test} ein.

Solche "benachbarten Werte" können die nächstmöglichen höheren und niedrigeren Werte in einer vordefinierten Liste möglicher AVD und/oder VVD-Werte sein oder Werte in einem vorgegebenen Abstand oberhalb und unterhalb der momentan eingestellten Werte AVDₐₖₜ und VVDₐₖₜ, oder systematisch ausgewählte Werte, die bei früheren Messungen besonders gute hämodynamische Ergebnisse erzielten oder zufällig ausgewählte Werte aus allen einstellbaren Werten in einem bestimmten vorgegebenen Intervall um die momentanen Werte für AVDₐₖₜ und VVDₐₖₜ.

Die hämodynamische Änderung ΔOpt für die ausgesuchten benachbarten Werte AVD_{Test} und VVD_{Test} wird anhand von aus einem oder mehreren Sensorsignalen abgeleiteten Parametern zur Erfassung von Volumengrößen (z. B. von der quadrupolaren intrakardialen Impedanzmessung, z. B. mit Stromeinspeisung auf der linken oder auf der rechten Herzseite), von Blutflussgrößen, von Blutdruckgrößen, Impedanzgrößen, elektrischen Größen oder von Abständen, Beschleunigungen oder Geschwindigkeiten, bewertet. Der Wert eines oder mehrerer dieser Parameter stellt den jeweiligen, den HDB charakterisierenden Wert dar.

Die Messungen zur Bestimmung der Werte der jeweiligen Parameter werden entweder einmalig durchgeführt oder mehrmals wiederholt und/oder es werden Mittelwerte gebildet.

Wenn das Messergebnis des den HDB charakterisierenden Parameters für benachbarte Testwerte AVD_{Test} und/oder VVD_{Test} besser ist für aktuelle Arbeitswerte AVDₐₖₜ und VVDₐₖₜ, werden die Testwerte als neue Arbeitswerte AVDₙₑᵤ und VVDₙₑᵤ übernommen und ein nächstes zu testendes Paar AVD_{Test} und VVD_{Test} wird in gleicher Messrichtung MR gewählt, in der die zuvor getesteten Testwerte AVD_{Test} und VVD_{Test} in Bezug auf die Ausgangswerte AVDₐₖₜ und VVDₐₖₜ lagen.

Wenn während eines Optimierungsschrittes - also eines Tests eines jeweiligen AVD_{Test} und VVD_{Test} - für Stabilitätsbedingungen, wie Frequenz oder Kontraktionsdynamik, ein Stabilitätskriterium nicht erfüllt ist, z. B. wenn sie ein vorgegebenes Intervall um die Werte bei Beginn des Optimierungsschrittes verlassen oder wenn ihre Streuung eine vorbestimmte Schwelle überschreitet, kann das zugehörige Messergebnis für den den HDB charakterisierenden Wert verworfen werden.

Im dargestellten Ausführungsbeispiel bestimmt der Herzstimulator 10 den jeweiligen HDB-Unterschied ΔOpt, indem er die Differenz zwischen zwei aus einer jeweiligen intrakardialen Impedanzmessung abgeleiteten Messwerten zu jeweils einem Wertepaar AVDₐₖₜ und VVDₐₖₜ sowie AVD_{Test} und VVD_{Test} bestimmt. Diese Messwerte können Werte solcher Parameter, wie maximale Impedanz oder mittlere Impedanz; Verhältnis von maximaler Impedanz zu mittlerer Impedanz oder Differenz aus maximaler Impedanz und mittlerer Impedanz sowie mittlerer Impedanz und minimaler Impedanz, maximale oder minimale Steilheit des Impedanz-Signals usw., sein.

Alternativ kann der Herzstimulator ausgebildet sein, die einen HDB charakterisierenden Werte aus Druck-Volumen-Diagrammen abzuleiten.

Um Testwerte für AVD und VVD zu testen ist der Schrittmacherzeitgeber ausgebildet, ausgehend von einem Arbeitspunkt AVDₐₖₜ und VVDₐₖₜ, zunächst den Wert der atrioventrikulären Übergangszeit zu verändern und danach, ebenfalls vom Arbeitspunkt ausgehend, den Wert der interventrikulären Übergangszeit zu verändern. Durch Auswertung des Optimierungskriteriums kann daraus die Richtung zum Optimum ermittelt und dementsprechend eine geeignete Messrichtung MR bestimmt werden. Beide Parameter (AVD und VVD) werden gemeinsam in Richtung dieses Optimums verändert. Dadurch kann für die Optimierung von AVD und VVD je ein voneinander unabhängiges Optimierungskriterium gewählt werden.

Für den Fall, dass die vorgegebenen Stabilitätsbedingungen erfüllt sind (z. B. die mittlere AVD Änderung pro Optimierungsschritt unterhalb eines vorgegebenen Schwellwertes liegt) und/oder nach vorgegebenen Zeitabständen oder ereignisgesteuert werden die Daten des momentanen Arbeitspunktes gespeichert. Diese Daten umfassen zum Beispiel die atrioventrikuläre Verzögerungszeit AVD, die interventrikuläre Verzögerungszeit VVD, die Herzfrequenz HF, die körperliche Belastung (die z. B. durch einen Ausgangswert des Aktivitätssensors 54 charakterisiert ist), Zeit, Stabilitätsbedingungen, Impedanzen, elektrische Signale oder hämodynamische Messwerte. Die Anzahl der Messwerte je AVD-VVD-Wertepaar ist fest vorgegeben, neue Messwerte führen zum Überschreiben älterer Messwerte oder werden in Gruppen sortiert, z. B. nach vordefinierten Herzfrequenzbereichen.

Zum Bestimmen geeigneter Testwerte und insbesondere zum Auffinden und Beibehalten eines globalen hämodynamischen Optimums ist der Schrittmacherzeitgeber ausgebildet, Ergebnisse vorangegangener Tests samt Messergebnis z. B. in einer Matrix zu speichern. Dieses Speichern der bereits erfassten Messwerte dient auch dazu, dass der Schrittmacherzeitgeber im Laufe der Zeit alle möglichen AVD/VVD-Kombinationen überprüft und damit sicher verhindert, dass er dauerhaft an Arbeitswerten für AVD und VVD festhält, die (nur) zu einem lokalen Maximum, nicht aber zu einem globalen Maximum des HDB, führen.

Hierzu ist der Schrittmacherzeitgeber ausgebildet, nicht jeweils nur einen Satz von nahe benachbarten Testwerten für AVD und VVD zu vermessen, sondern eine Auswahl "weiter entfernter" AVD/VVD-Kombinationen, was zur Vermeidung lokaler Maxima beiträgt, zum Beispiel:
- Solche AVD/VVD-Kombinationen, zu denen der den HDB charakterisierende Wert noch nicht gemessen wurde oder dessen letzte Vermessung am längsten zurückliegt;
- solche AVD/VVD-Kombinationen, die bei einem vergleichbaren Aktivitätsgrad (ähnlicher Herzfrequenz, gleicher atrialer Ereignistyp ähnlichem Aktivitätssensorausgangswert, usw.) aufgezeichnet wurden;
- solche AVD/VVD-Kombinationen, die eine maximale hämodynamische Bewertung (mit oder ohne übereinstimmenden Aktivitätsgrad) ergeben.

Auch kann der Schrittmacherzeitgeber zur stochastischen Auswahl von zu testenden AVD/VVD-Kombinationen ausgebildet sein, die innerhalb einer vorgegebenen Umgebung liegen, um so vorzugsweise noch fehlende Einträge zu ergänzen.

Die Auswahl von zu testenden AVD/VVD-Kombinationen kann auch auf Verfahren beruhen, die auf der Auswertung intrakardialer Elektrokardiogramme (IEGM) basieren.

Außerdem ist der Schrittmacherzeitgeber ausgebildet, in vorgegebenen, ggf. einstellbaren Zeitabständen oder wenn ein IEGM-Kriterium erfüllt ist, einen neuen AVD/VVD-Arbeitspunkt, wie oben beschrieben, zu ermitteln. Von dort aus wird wiederum die obige Optimierung angewandt.

Alternativ kann der Schrittmacherzeitgeber ausgebildet sein, in vorgegebenen, ggf. einstellbaren, Zeitabständen oder wenn ein Kriterium aus Änderungen von zum Beispiel Herzfrequenz, Kontraktionsdynamik, Aktivitätsgrad oder Tageszeit erfüllt ist, einen neuen AVD/VVD-Arbeitspunkt, wie oben beschrieben, zu bestimmen. Von diesem ausgehend wird wiederum die zuvor beschriebene Optimierung angewandt.

Der Schrittmacherzeitgeber ist außerdem ausgebildet, intrinsische atrioventrikuläre Überleitungen zuzulassen und durch entsprechende Variationen der Arbeitswerte für AVD und VVD zu fördern. Intrinsische atrioventrikuläre Überleitungen sind solche, bei denen es zu einer natürlichen, nicht stimulierten Kontraktion von linkem und oder rechtem Ventrikel aufgrund natürlicher Reizleitung im Herzen kommt.

Der Schrittmacherzeitgeber überprüft, ob das hämodynamische Signal oder die hämodynamischen Signale bestimmten Qualitätskriterien genügen. Wenn dies nicht der Fall ist, wendet er automatisch eine alternative Optimierungsmethode an.

Für den Fall, dass keine hämodynamische Messung in ausreichender Qualität möglich ist, geht der Schrittmacherzeitgeber automatisch auf normale (nicht optimierte) AVD/VVD-Dynamik-Parameter über.

Die Funktionsweise des Schrittmacherzeitgebers kann wie folgt zusammengefasst werden: Von einem aktuellen Arbeitspunkt ausgehend (Kreis in Figur 4) werden die Nachbar-Werte gemessen, um sich schrittweise dem Optimum zu nähern (Pfeile). In regelmäßigen Abständen wird ein zufällig gewählter Satz von Einstellungen (Quadrate) gemessen. Wie auch die Matrixvariation, dient diese Methode dazu, einem lokalen Maximum (in Figur 4 z. B. VVD20, AVD80) "entkommen".

Um die Funktionsweise der Schrittmachsteuerung und des Schrittmacherzeitgebers umprogrammieren zu können oder geeignete Werte für Stimulationsparameter vorgeben zu können, ist die Schrittmachersteuerung mit einer Telemetrieeinheit TEL 62 verbunden. Über diese können auch im Speicher 60 gespeicherte Werte, z. B. Messwerte wie den jeweiligen HDB für verschiedene AVD und VVD Werte, an ein zentrales Servicecenter übertragen werden.

## Patentansprüche

1. Biventrikulärer Herzstimulator mit
- einer rechtsventrikulären Stimulationseinheit (42), die mit einer rechtsventrikulären Stimulationselektrode (28) verbunden oder zu verbinden und ausgebildet ist, auf ein rechtsventrikuläres Triggersignal hin wenigstens einen Stimulationsimpuls über die rechtsventrikuläre Elektrode abzugeben,
- einer linksventrikulären Stimutationseinheit (44), die mit einer linksventrikulären Stimulationselektrode (32) verbunden oder zu verbinden und ausgebildet ist, auf ein linksventrikuläres Triggersignal hin wenigstens einen Stimulationsimpuls über die linksventrikuläre Elektrode abzugeben,
- einem Schrittmacherzeitgeber, der mit der rechtsventrikulären Stimulationseinheit und der linksventrikulären Stimulationseinheit verbunden und ausgebildet ist, rechtsventrikuläre Triggersignale und linksventrikuläre Triggersignale am Ende einer atrioventrikulären Verzögerungszeit (AVD) bzw. einer interventrikulären Verzögerungszeit (VVD) abzugeben, und
- einer Erfassungseinheit für einen hämodynamischen Benefit (HDB), die den HDB durch Auswertung eines hämodynamischen Sensors quantifiziert,
wobei der Schrittmacherzeitgeber mit einem Speicher (60) für einen jeweiligen aktuellen Wert für die atrioventrikuläre Verzögerungszeit (AVDakt) und die interventrikuläre Verzögerungszeit (VVDakt) verbunden und ausgebildet ist, zu bestimmten Zeitpunkten wenigstens jeweils ein rechtsventrikuläres Triggersignal und ein linksventrikuläres Triggersignal, basierend auf von den aktuellen Werten für die atrioventrikuläre Verzögerungszeit (AVDakt) und die interventrikuläre Verzögerungszeit (VVDakt) abweichenden Testwerten, für die atrioventrikuläre Verzögerungszeit (AVDTest) und die interventrikuläre Verzögerungszeit (VVDTest) auszulösen **dadurch gekennzeichnet, dass** der Schrittmacherzeitgeber dazu ausgebildet ist, den zu einem jeweiligen Wertepaar von AVDTest und VVDTest ermittelten HDB nach Art einer Matrix den beiden Werten für AVDTest und VVDTest zugeordnet zu speichern und
von einem durch aktuelle Werte der atrioventrikulären Verzögerungszeit (AVD_{Akt}) und der interventrikulären Verzögerungszeit (VVD_{Akt}) definierten Arbeitspunkt ausgehend Testwerte für die atrioventrikuläre Verzögerungszeit und die interventrikuläre Verzögerungszeit zu bestimmen, die in einer vom Arbeitspunkt ausgehenden Messrichtung (MR) liegen, wobei der Schrittmacherzeitgeber weiter dazu ausgebildet ist, die Messrichtung durch Auswertung gespeicherter Werte für die atrioventrikuläre und die interventrikuläre Verzögerungszeit sowie zugehörige, den jeweiligen HDB charakterisierende Werte zu bestimmen..

2. Biventrikulärer Herzstimulator nach Anspruch 1, **dadurch gekennzeichnet, dass** der Schrittmacherzeitgeber dazu ausgebildet ist, zur Bestimmung geeigneter Testwerte für die atrioventrikuläre Verzögerungszeit (AVD_{Test}) und die interventrikuläre Verzögerungszeit (VVD_{Test}) gespeicherte Werte für mögliche atrioventrikuläre Verzögerungszeiten und interventrikuläre Verzögerungszeiten samt zugehörigem, den HDB charakterisierenden Wert auszuwerten.

3. Biventrikulärer Herzstimulator nach Anspruch 2, **dadurch gekennzeichnet, dass** der Schrittmacherzeitgeber in diesem Zusammenhang dazu ausgebildet ist, solche Testwerte für die atrioventrikuläre Verzögerungszeit und die interventrikuläre Verzögerungszeit zu wählen, für den noch kein den HDB charakterisierender Wert gespeichert ist.

4. Biventrikulärer Herzstimulator nach Anspruch 2, **dadurch gekennzeichnet, dass** der Schrittmacherzeitgeber dazu ausgebildet ist, solche Testwerte für die atrioventrikuläre Verzögerungszeit und die interventrikuläre Verzögerungszeit auszuwählen, für die ein jeweils zugehöriger, den HDB charakterisierender Wert zu einem Zeitpunkt bestimmt wurde, der länger als ein vorgegebenes Zeitmaß zurückliegt.

5. Biventrikulärer Herzstimulator nach Anspruch 2, **dadurch gekennzeichnet, dass** der Schrittmacherzeitgeber dazu ausgebildet ist, solche Testwerte für die atrioventrikuläre Verzögerungszeit und die interventrikuläre Verzögerungszeit auszuwählen, für die ein HDB gespeichert ist, der größer ist als der aktuelle Wert und nicht in dessen direkter Nachbarschaft platziert ist.

6. Biventrikulärer Herzstimulator nach Anspruch 1, **dadurch gekennzeichnet, dass** der Schrittmacherzeitgeber dazu ausgebildet ist, eine korrekte Messrichtung (MR) aus Trenddaten der letzten Arbeitspunkte zu Bestimmen wenn die Genauigkeit der Einzelmessungen dazu nicht ausreicht.

7. Biventrikulärer Herzstimulator nach Anspruch 1, **dadurch gekennzeichnet, dass** der Schrittmacherzeitgeber ausgebildet ist, immer dann, wenn eine mit jeweils aktuell getesteten Testwerten für die atrioventrikuläre Verzögerungszeit (AVD_{Test}) und die interventrikuläre Verzögerungszeit (VVD_{Test}) verbundene Verbesserung des HDB ein vorgegebenes Mindestmaß überschreitet, die Testwerte für die atrioventrikuläre Verzögerungszeit und die interventrikuläre Verzögerungszeit zu neuen Arbeitswerten der atrioventrikulären Verzögerungszeit (AVDₙₑᵤ) und der interventrikulären Verzögerungszeit (VVDₙₑᵤ) für die weitere reguläre Stimulation zu machen.

8. Biventrikulärer Herzstimulator nach Anspruch 1, **dadurch gekennzeichnet, dass** der Schrittmacherzeitgeber dazu ausgebildet ist, Testwerte für die atrioventrikuläre Verzögerungszeit und die interventrikuläre Verzögerungszeit zu verwerfen, sofern diese oder der mit ihnen verbundene, den HDB charakterisierende Wert vorgegebene Qualitätsbedingungen nicht erfüllen.

9. Biventrikulärer Herzstimulator nach Anspruch 8, **dadurch gekennzeichnet, dass** der Schrittmacherzeitgeber ausgebildet ist, für den Fall, dass die Qualitätsbedingungen nicht erfüllt sind, automatisch auf vorgegebene Werte für AVD und VVD überzugehen.

10. Biventrikulärer Herzstimulator nach Anspruch 1, **dadurch gekennzeichnet, dass** der Schrittmacherzeitgeber ausgebildet ist, intrinsische atrioventrikuläre Überleitungen zuzulassen und durch entsprechende Variationen der Arbeitswerte für AVD und VVD zu fördern.

11. Biventrikulärer Herzstimulator nach Anspruch 1, **dadurch gekennzeichnet, dass** der Schrittmacherzeitgeber ausgebildet ist, in wiederkehrenden Zeitintervallen zufällig bestimmte Werte für AVD_{Test} und VVD_{Test} anzuwenden.

## Claims

1. A biventricular cardiac stimulator, comprising
- a right ventricular stimulation unit (42), which is connected, or is to be connected, to a right ventricular stimulation electrode (28) and designed to deliver at least one stimulation pulse via the right ventricular electrode in response to a right ventricular trigger signal,
- a left ventricular stimulation unit (44), which is connected, or is to be connected, to a left ventricular stimulation electrode (32) and designed to deliver at least one stimulation pulse via the left ventricular electrode in response to a left ventricular trigger signal,
- a pacemaker timer, which is connected to the right ventricular stimulation unit and the left ventricular stimulation unit and designed to deliver right ventricular trigger signals and left ventricular trigger signals at the end of an atrioventricular delay (AVD) or an interventricular delay (VVD), and
- a detection unit for a hemodynamic benefit (HDB), which quantifies the HDB by evaluating a hemodynamic sensor,
the pacemaker timer being connected to a memory (60) for a respective current value for the atrioventricular delay (AVD_{act}) and for the interventricular delay (VVD_{act}) and designed to trigger, at defined times, at least one right ventricular trigger signal and a left ventricular trigger signal, based on test values that deviate from the current values for the atrioventricular delay (AVD_{act}) and for the interventricular delay (VVD_{act}), for the atrioventricular delay (AVD_{Test}) and the interventricular delay (VVD_{Test}), **characterized in that** the pacemaker timer is designed to store the HDB determined for a respective value pair of AVD_{Test} and VVD_{Test} in the form of a matrix in such a way that the HDB is associated with the two values for AVD_{Test} and VVD_{Test}, and, proceeding from a working point that is defined by the current values of the atrioventricular delay (AVD_{act}) and the interventricular delay (VVD_{act}), to determine test values for the atrioventricular delay and the interventricular delay which are located in a measuring direction (MR) extending from the working point, wherein the pacemaker timer is further designed to determine the measuring direction by evaluating stored values for the atrioventricular delay and the interventricular delay as well as associated values characterizing the respective HDB.

2. The biventricular cardiac stimulator according to claim 1, **characterized in that** the pacemaker timer is designed to evaluate values, which are stored for determining suitable test values for the atrioventricular delay (AVD_{Test}) and the interventricular delay (VVD_{Test}), for potential atrioventricular delays and interventricular delays, including the associated value characterizing the HDB.

3. The biventricular cardiac stimulator according to claim 2, **characterized in that** the pacemaker timer in this context is designed to select such test values for the atrioventricular delay and the interventricular delay for which no value characterizing the HDB is stored yet.

4. The biventricular cardiac stimulator according to claim 2, **characterized in that** the pacemaker timer is designed to select such test values for the atrioventricular delay and the interventricular delay for which a respective associated value characterizing the HDB was determined at a time which dates back longer than a predetermined unit of time.

5. The biventricular cardiac stimulator according to claim 2, **characterized in that** the pacemaker timer is designed to select such test values for the atrioventricular delay and the interventricular delay for which an HDB is stored which is greater than the current value and cannot be found in direct vicinity thereto.

6. The biventricular cardiac stimulator according to claim 1, **characterized in that** the pacemaker timer is designed to determine a correct measuring direction (MR) from trend data of the last working points if the accuracy of the individual measurements is not sufficient to do so.

7. The biventricular cardiac stimulator according to claim 1, **characterized in that** the pacemaker timer is designed to turn the test values for the atrioventricular delay and the interventricular delay into new working values of the atrioventricular delay (AVD_{new}) and interventricular delay (VVD_{new}) for the continued regular stimulation every time when an improvement in the HDB that is associated with respective currently tested test values for the atrioventricular delay (AVD_{Test}) and the interventricular delay (VVD_{Test}) exceeds a predetermined minimum amount.

8. The biventricular cardiac stimulator according to claim 1, **characterized in that** the pacemaker timer is designed to discard test values for the atrioventricular delay and the interventricular delay if these, or the value associated with them which characterizes the HDB, do not meet predetermined quality conditions.

9. The biventricular cardiac stimulator according to claim 8, **characterized in that** the pacemaker timer is designed to automatically switch to predetermined values for AVD and VVD for the event that the quality conditions are not met.

10. The biventricular cardiac stimulator according to claim 1, **characterized in that** the pacemaker timer is designed to allow intrinsic atrioventricular conduction and to promote such conduction through corresponding variations of the working values for AVD and VVD.

11. The biventricular cardiac stimulator according to claim 1, **characterized in that** the pacemaker timer is designed to apply values for AVD_{Test} and VVD_{Test} which were randomly determined at recurring time intervals.

## Revendications

1. Stimulateur cardiaque biventriculaire avec
- une unité de stimulation de ventricule droit (42), qui est reliée ou destinée à être reliée à une électrode de stimulation de ventricule droit (28), et conçue pour délivrer au moins une impulsion de stimulation, par le biais de l'électrode de ventricule droit, en réponse à un signal de déclenchement de ventricule droit,
- une unité de stimulation de ventricule gauche (44), qui est reliée ou destinée à être reliée à une électrode de stimulation de ventricule gauche (32), et conçue pour délivrer au moins une impulsion de stimulation, par le biais de l'électrode de ventricule gauche, en réponse à un signal de déclenchement de ventricule gauche,
- une horloge de stimulateur cardiaque, reliée à l'unité de stimulation de ventricule droit et à l'unité de stimulation de ventricule gauche, et conçue pour délivrer des signaux de déclenchement de ventricule droit et des signaux de déclenchement de ventricule gauche, à la fin d'un temps de retardement atrio-ventriculaire (AVD) ou d'un temps de retardement interventriculaire (VVD), et
- une unité de détection pour un bénéfice hémodynamique (HDB), quantifiant le HDB par l'évaluation d'un capteur hémodynamique,
dans lequel l'horloge de stimulateur cardiaque est reliée à une mémoire destinée à une valeur actuelle pour le temps de retardement atrio-ventriculaire (AVDakt) et le temps de retardement interventriculaire (VVDakt), et conçue pour déclencher, à des instants déterminés, au moins respectivement un signal de déclenchement de ventricule droit et un signal de déclenchement de ventricule gauche, sur la base des valeurs de test pour le temps de retardement atrio-ventriculaire (AVDtest) et le temps de retardement inter-ventriculaire (VVDtest), qui dévient des valeurs actuelles pour le temps de retardement atrio-ventriculaire (AVDakt) et le temps de retardement inter-ventriculaire (VVDakt), **caractérisé en ce que** l'horloge de stimulateur cardiaque est conçue pour enregistrer le HDB fourni respectivement pour une paire de valeurs AVDtest et VVDtest, à la façon d'une matrice, en l'attribuant aux deux valeurs AVDtest et VVDtest, et pour déterminer, à partir d'un point de travail défini par des valeurs actuelles du temps de retardement atrio-ventriculaire (AVDakt) et du temps de retardement inter-ventriculaire (VVDakt), des valeurs de test pour le temps de retardement atrio-ventriculaire et le temps de retardement inter-ventriculaire, qui se trouvent dans une direction de mesure (MR) partant du point de travail, l'horloge de stimulateur cardiaque étant en outre conçue pour déterminer la direction de mesure par l'évaluation de valeurs enregistrées pour le temps de retardement atrio-ventriculaire et inter-ventriculaire, ainsi que des valeurs correspondantes, caractérisant le HDB respectif.

2. Stimulateur cardiaque biventriculaire selon la revendication 1, **caractérisé en ce que** l'horloge de stimulateur cardiaque est conçue pour évaluer des valeurs enregistrées pour la détermination de valeurs de test appropriées pour le temps de retardement atrio-ventriculaire (AVDtest) et le temps de retardement interventriculaire (VVDtest), pour des temps de retardement atrio-ventriculaire possibles et des temps de retardement inter-ventriculaire possibles, ensemble avec la valeur caractérisant le HDB.

3. Stimulateur cardiaque biventriculaire selon la revendication 2, **caractérisé en ce que** l'horloge de stimulateur cardiaque est, dans ce contexte, conçue pour sélectionner des valeurs de test pour le temps de retardement atrio-ventriculaire et le temps de retardement inter-ventriculaire, pour lesquelles aucune valeur caractérisant un HDB n'a encore été enregistrée.

4. Stimulateur cardiaque biventriculaire selon la revendication 2, **caractérisé en ce que** l'horloge de stimulateur cardiaque est conçue pour sélectionner des valeurs de test pour le temps de retardement atrio-ventriculaire et le temps de retardement inter-ventriculaire, pour lesquelles une valeur caractérisant le HDB a été déterminée à un instant antérieur à une mesure prédéterminée.

5. Stimulateur cardiaque biventriculaire selon la revendication 2, **caractérisé en ce que** l'horloge de stimulateur cardiaque est conçue pour sélectionner des valeurs de test pour le temps de retardement atrio-ventriculaire et le temps de retardement inter-ventriculaire, pour lesquelles un HDB supérieur à la valeur actuelle et placé ailleurs qu'à sa proximité immédiate a été enregistré.

6. Stimulateur cardiaque biventriculaire selon la revendication 1, **caractérisé en ce que** l'horloge de stimulateur cardiaque est conçue pour déterminer une direction de mesure (DM) correcte à partir des données de tendance des derniers points de travail, lorsque l'exactitude des mesures individuelles n'est pas suffisante à cette fin.

7. Stimulateur cardiaque biventriculaire selon la revendication 1, **caractérisé en ce que** l'horloge de stimulateur cardiaque est conçue pour transformer les valeurs de test pour le temps de retardement atrio-ventriculaire et le temps de retardement inter-ventriculaire en nouvelles valeurs de travail du temps de retardement atrio-ventriculaire (AVDneu) et du temps de retardement interventriculaire (VVDneu), pour la suite de la stimulation régulière, à chaque fois qu'une amélioration du HDB liée à des valeurs de test actuellement testées pour le temps de retardement atrio-ventriculaire (AVDtest) et le temps de retardement inter-ventriculaire (VVDtest) dépasse une mesure minimum prédéterminée.

8. Stimulateur cardiaque biventriculaire selon la revendication 1, **caractérisé en ce que** l'horloge de stimulateur cardiaque est conçue pour rejeter des valeurs de test pour le temps de retardement atrio-ventriculaire et le temps de retardement inter-ventriculaire, lorsque celles-ci ou la valeur caractérisant le HDB correspondant ne répondent pas aux conditions de qualité prédéfinies.

9. Stimulateur cardiaque biventriculaire selon la revendication 8, **caractérisé en ce que** l'horloge de stimulateur cardiaque est conçue pour passer automatiquement à des valeurs prédéterminées pour AVD et VVD lorsque les conditions de qualité ne sont pas respectées.

10. Stimulateur cardiaque biventriculaire selon la revendication 1, **caractérisé en ce que** l'horloge de stimulateur cardiaque est conçue pour permettre des conductions atrio-ventriculaires intrinsèques et pour les favoriser par des variations correspondantes des valeurs de travail pour AVD et VVD.

11. Stimulateur cardiaque biventriculaire selon la revendication 1, **caractérisé en ce que** l'horloge de stimulateur cardiaque est conçue pour appliquer des valeurs déterminées au hasard pour AVDtest et VVDtest, à des intervalles de temps périodiques.
